# EUROPEAN PATENT APPLICATION

(11) **EP 0 853 088 A2**
(43) Date of publication of application: **15.07.1998**
(21) Application number: 98100535.8
(22) Date of filing: 14.01.1998
(51) Int. Cl.: C07K 14/47, C12N 9/02

(54) **A thioredoxin variant and a factor comprising said variant to enhance AP-1 transcriptional activity**

(30) Priority: 14.01.1997 JP 4489/97
(71) Applicant: ORIENTAL YEAST CO., LTD., Tokyo (JP); Yodoi, Junji, Sakyo-ku Kyoto-Shi Kyoto-fu (JP)
(72) Inventor: Yodoi, Junji, Kyoto-shi, Kyoto (JP)
(74) Representative: Kolb, Helga, Dr. Dipl.-Chem.

(57) **Abstract**

The present invention has an object to provide a thioredoxin variant which is stable under non-reducing conditions and a factor comprising said thioredoxin and Ref-1 to enhance AP-1 transcriptional activity.

The thioredoxin variant of the present invention is characterized in that at least one or all Cys residues are substituted by other amino acid residues except that Cys residues in the active center remain unmodified. The factor of the present invention to enhance AP-1 transcriptional activity comprises said thioredoxin and Ref-1 associated with each other via an S-S bond.

## Description

The present invention relates to thioredoxin variants which are stable in non-reducing conditions and in even in oxidizing conditions.

The present invention also relates to a factor comprising said variant to enhance AP-1 transcriptional activity.

In recent years, a series of evidences have suggested that intracellular events including proliferation and apotosis are controlled by intracellular redox state (Pahl, H.L. & Baeuerle, P.A. (1994) BioAssays 16, 497-502). Particularly, in vitro gel shift assays have successively revealed transcrip-tion factors which are controlled by thiol-redox regulation mechanism (Matthews, J.R., Wakasugi, N., Virelizier, J.L., Yodoi, J. & Hay, R.T. (1992) Nucleic Acids Res. 20, 3821-30; Abate, C., Patel, L., Rauscher, F.J.d. & Curran, T. (1990) Science 249, 1157-1161). They include known transcription factors such as AP-1, NF-κB and other members of the Dorsal/Rel family, Myb and Ets, etc. As to the control of the activity of these transcription factors, chemical reagents such as 2-ME or DTT are known to activate the DNA-binding ability of these transcription factors, but it has not been clarified yet which molecules intracellularly act as a redox regulator.

Thioredoxin (hereinafter referred to as "TRX") has attracted attention as a likely candidate for an intracellular redox regulator. TRX is a small protein which is ubiquitous in living bodies and has a site with two cysteines -Cys-Gly-Pro-Cys- in its active center (Laurent, T.C., Moore, E.C., & Reichard, P. (1964) J. Biol. Chem. 239, 3436-3444; Holmgren, A. (1989) Ann. Rev. Biochem. 54, 237-271; Holmgren, A. (1989) J. Biol. Chem. 264, 13963-1366; Buchanan, B.B., Schurmann, P., Decottignies, P. & Lozano, R.M. (1994) Arch. Biochem. Biophys. 314, 257-60), and thus forms a pleiotropic cellular factor which participates in redox reactions via reversible oxidation of dithiol in the active center. TRX exists in both reduced and oxidized forms and seems to play an important role in the control of intracellular events including gene expression. It is also known as an adult T-cell leukemia-derived factor (ADF) involved in HTLV-I leukemogenesis. TRX functions intracellularly (Matthews, J.R. at al., 1992, supra.; Okamoto, T., Ogiwara, H., Hayashi, T., Mitsui, A., Kawabe, T. & Yodoi J. (1992) Int. Immunol. 4, 811-9) and extra-cellularly. One of its intracellular functions is known to promote protein-nucleotide interactions.

NF-κB is a transcription factor which seems to participate in the activation of the transcription of the κL chain gene (Sen, R. & D. Baltimore (1986) Cell 46, 705-716). NF-κB comprises homo-dimer or hetero-dimer of members of rel family including c-rel, p65, p50, p49 and relB. In vitro and in vivo experiments showed that TRX enhanced the DNA-binding activity and transctiptional activity of the p50 subunit of NF-κB by reducing the Cys residue at 62 of p50. Recently, a direct physical association between TRX and an oligopeptide from NF-κB p50 was revealed by in vitro experiments by NMR. However, a direct in vivo association between them has not been confirmed.

It has also been suggested that another transcription factor, AP-1, is also controlled via redox. AP-1 is a gene product of a proto-oncogene (c-jun) as an intracellular prototype of avian sarcoma virus 17 (v-jun). The c-jun gene can generate two kinds of mRNAs, which are about 3.2kb and 2.6kb and encodes a nucleic protein of about 40kD. The entire nucleic acid sequence of c-jun gene and the amino acid sequence of AP-1 protein encoded by the gene are disclosed in Hattori, K., Angel, P., Le Beau, M.M. & Karin, M. 1988, Proc. Natl. Acad. Sci. U.S.A. 85(23), 9148-9152, for example. The AP-1 protein forms a homo-dimer which binds to a specific DNA sequence (TGACTCA) and also to a certain cAMP response element (CRE), thus expressing its transcriptional control activity. Alternatively, AP-1 forms a stable dimer with a fos-related gene product Fos via a leucine-zipper structure to remarkably enhance its transcriptional activation (Dictionary of Terminology of Immunology, 3rd ed., by Tomio TADA et al., December 1, 1993). Various antioxidants are known to strongly activate the DNA-binding ability and transcriptional activation of AP-1 (Pahl, H.L. et al., (1994) supra., etc.), thus indicating that the ability of AP-1 to activate transcription is controlled via redox.

However, AP-1 is not a direct substrate for TRX, unlike NF-κB (Xanthoudakis, S., Miao, G., Wang, F., Pan, Y.C. & Curran, T. (1992) EMBO J. 11, 3323-35). Moreover, the sequence surrounding the Cysteine of AP-1 is entirely different from that of NF-κB (Okuno, H., Akahori, A., Sato, H., Xahthoudakis, S., Curran, T. & Iba, H. (1993) Oncogene 8, 695-701; Ng, L., Forrest, D. & Curran, T. (1993) Nucleic Acids Res. 21, 5831-5837). Therefore, the transcription factor AP-1 seems to have a redox regulation mechanism which requires the presence of molecules other than TRX, unlike NF-κB. Such molecules include redox factor 1 (hereinafter referred to as "Ref-1") (Xanthoudakis, S. & Curran, T. (1992) EMBO J 11, 653-65; Xanthoudakis, S., Miao, G.G. & Curran, T. (1994) Proc. Natl. Acad. Sci., USA 91, 23-7). Ref-1 is a protein which was identified in a cell-free system as one of the factors restoring the AP-1-DNA binding and found to be identical with AP-1 endonuclease.

Thus, it has been known that the redox regulation of AP-1 requires the presence of two factors, i.e. TRX and Ref-1. However, no detailed mechanism of promoting the transcriptional activity of AP-1 has been explained, and no report has appeared prior to the present invention showing whether TRX and Ref-1 directly associate with each other or an additional intervening factor is required for their interaction, etc. Moreover, TRX had difficulty in handling because it has a plurality of cysteine residues in its molecules so that it tends to precipitate by forming a multimer. Therefore, no useful AP-1 transcriptional activity enhancer has been obtained despite of a great need therefor.

An object of the present invention is to provide TRX which is stable under non-reducing conditions, by substituting at least one or all Cys residues by other amino acid residues except that Cys residues in the active center of TRX remain unmodified.

Another object of the present invention is to provide a factor to enhance AP-1 transcriptional activity wherein said factor comprises said TRX and Ref-1 associated via an S-S bond.

FIG. 1 shows a schematic representation of mutant TRX constructs used in the examples described herein.

FIG. 2 shows effects of the transient expression of TRX and/or Ref-1 on the activation of AP-1 in PMA-treated HeLa cells.

FIG. 3 shows Western blot analysis of various TRX complexes using an anti-TRX antibody.

FIG. 4 shows Western blot analysis of various TRX and Ref-1 complexes using an anti-Ref-1 antibody.

FIG. 5 shows Western blot analysis of various TRX and Ref-1 complexes using an anti-TRX antibody.

FIG. 6 shows a physical interaction between the wild-type TRX and Ref-1 by mammalian two-hybrid assay in COS7 cells.

FIG. 7 shows a physical interaction between TRX mutants and Ref-1 by mammalian two-hybrid assay in COS7 cells.

As a result of profound study to solve the above problems, we found that TRX directly associates with Ref-1 to promote the AP-1 transcriptional activity and that Cys residues in the active center of TRX are essential for said association.

Thus, we provided TRX which is stable under non-reducing conditions, by substituting at least one or all Cys residues by other amino acid residues except that the Cys residues constituting the active center remain unmodified. We also could provide a factor to enhance AP-1 transcriptional activity wherein said factor comprises said variant TRX and Ref-1 associated via an S-S bond. The present invention is explained in detail below.

TRX in the present invention includes proteins which have a site with two cysteines -Cys-Gly-Pro-Cys- in its active center and thus can form a pleiotropic cellular factor which participates in redox reactions via reversible oxidation of dithiol in the active center.

As mentioned before, TRX is ubiquitous in living bodies, and TRX of the present invention is not limited to any particular type of TRX of a certain species. The amino acid sequence and base sequence of TRX are known and described in a plurality of documents (Laurent, T.C. et al., 1964, supra.; Holmgren, A. Ann. Rev. Biochem. 1989, supra.; Holmgren, A., J. Biol. Chem., 1989, supra.; Buchanan, B.B. et al., 1994, supra.). For example, human and murine TRX are described in Tagaya, Y., Maeda, A., Kondo, N., Matsui, H., Hamuro, J., Brown, N., Arai, K., Yokota, T. & Yodoi, J. (1989) EMBO J.8 (3), 757-764. The TRX of human is preferable.

Ref-1 of the present invention includes a protein which functions as AP-1 endnuclease and can restore the AP-1-DNA binding.

As with TRX, Ref-1 is ubiquitous in living bodies, and Ref-1 of the present invention is not limited to any particular type of Ref-1 of a certain species. The amino acid sequence and base sequence of Ref-1 are known and described in a plurality of documents. For example, human Ref-1 is described in Xanthoudakis, S., Miao, G., Wang, F., Pan, Y.C. & Curran, T. (1992) EMBO J. 11(9), 3323-3335, and murine Ref-1 is described in Smas, C.M., Green, D. & Sul, H.S. (1994) Biochemistry 33(31), 9257-9265. The Ref-1 of human is preferable.

Enhancement of AP-1 transcriptional activity by the factor of the present invention comprising the variant TRX and Ref-1 can be detected as described in Promega Technical Bulletin, No.101, 1990.

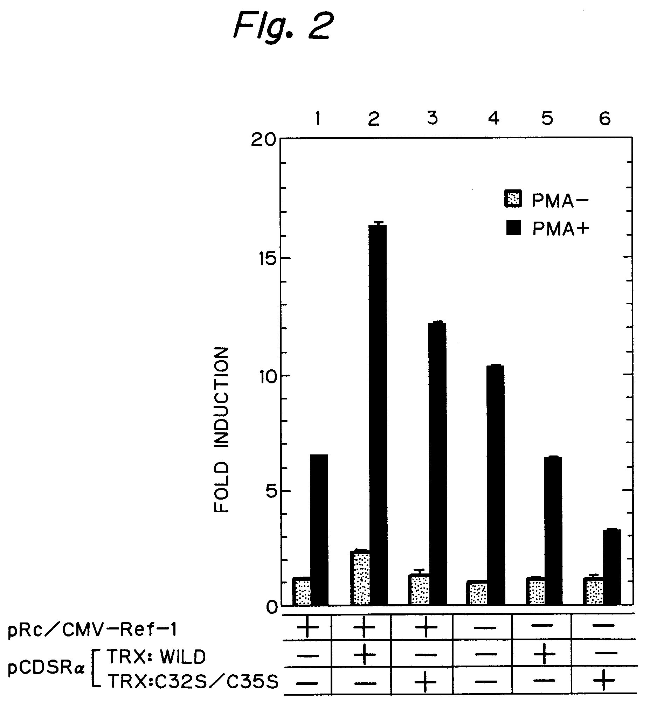

We examined the effects of overexpression of TRX and/or Ref-1 on the transcriptional activity of AP-1 in vivo (Example 1). As a result, co-expression of the wild-type TRX and Ref-1 in HeLa cells treated with a transcriptional activity inducer of AP-1, 12-O-tetradecanoylphorbol 13-acetate (PMA), showed a transcriptional activity about four times higher than a control (Fig. 2, line 2). When the wild-type TRX or Ref-1 was expressed alone, the transcriptional activity was about 1.5 to 2.0 times higher than the control (Fig. 2, lanes 5 and 1). Therefore, TRX and Ref-1 seem to cooperatively promote the activity of AP-1.

This is also supported by intracellular localization of TRX and Ref-1. TRX exists in cytoplasm before cells are treated with a transcriptional activity inducer such as PMA, and moves into nuclei after treatment. However, Ref-1 exists in nuclei both before and after treatment (Example 2). Thus, both TRX and Ref-1 are localized in nuclei when cells are treated with a transcriptional activity inducer.

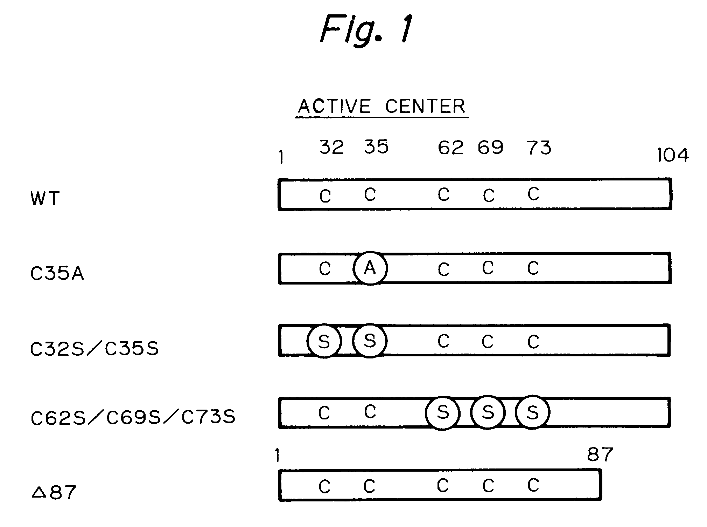

We further examined whether TRX and Ref-1 directly associate or any intervening factor is required for promoting the transcriptional activity of AP-1. TRX protein has several cysteine residues in its molecules. For example, human TRX has five cysteine residues at the 32-, 35-, 62-, 69- and 73-position, as shown in Fig. 1. Among them, the site containing two cysteines at the 32- and 35 positions -Cys-Gly-Pro-Cys- is known to be a redox active site (Laurent, T.C. et al., 1964, supra.; Holmgren, A., Ann. Rev. Biochem. 1989, supra.; Holmgren, A., J. Biol. Chem. 1989, supra.; Buchanan, B.B. et al., 1994, supra.). We prepared TRX mutants by modifying these cysteine residues and used a crosslinking reagent which converts SH groups into an -S-S-bond by Cys oxidation to reveal that TRX and Ref-1 directly associate with each other and that the association between TRX and Ref-1 particularly involves Cys32 in the active center.

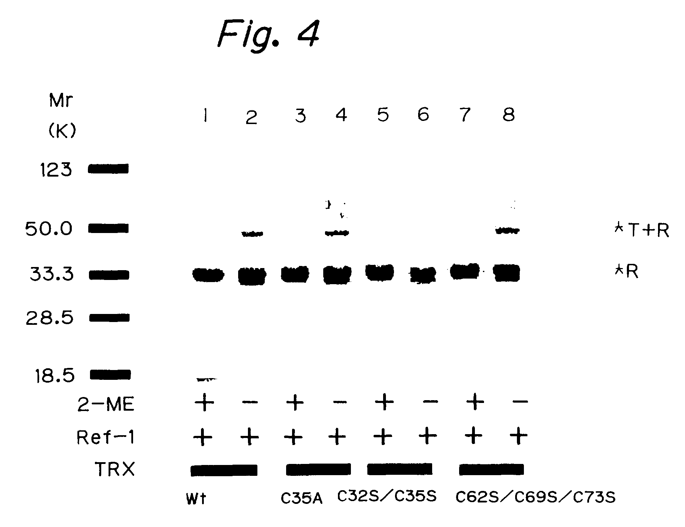

Specifically, the wild-type TRX and Ref-1 were mixed under oxidizing conditions to form a wild-type TRX-Ref-1 complex, as evidenced by an appearance of a band which reacts with wild-type TRX antibodies and an Ref-1 antibody at about 50 kDa corresponding to the sum of the molecular weights of TRX and Ref-1. TRX mutants, TRX^{C35A} (substitution of alanine for Cys35 of human TRX), TRX^{C32S/C35S} (substitution of serine for each of Cys32 and Cys35 of human TRX) and TRX^{C62S/C69S/C73S} (substitution of serine for each of Cys62, Cys69 and Cys73 of human TRX) were also prepared and examined for their association with Ref-1 (Example 3). TRX^{C35A} and TRX^{C62S/C69S/C73S} associated with Ref-1 in the same way as the wild-type (Fig. 4, lanes 4 and 8). However, TRX^{C32S/C35S} did not form any conjugate with Ref-1 (Fig. 4, lane 6).

These results demonstrate that TRX directly associates with Ref-1 to control the transcriptional activity of AP-1, i.e. TRX is one of proton donors of Ref-1. It was also found that the association between TRX and Ref-1 involves Cys32 and Cys35 which constitute the active center of TRX, particularly Cys32.

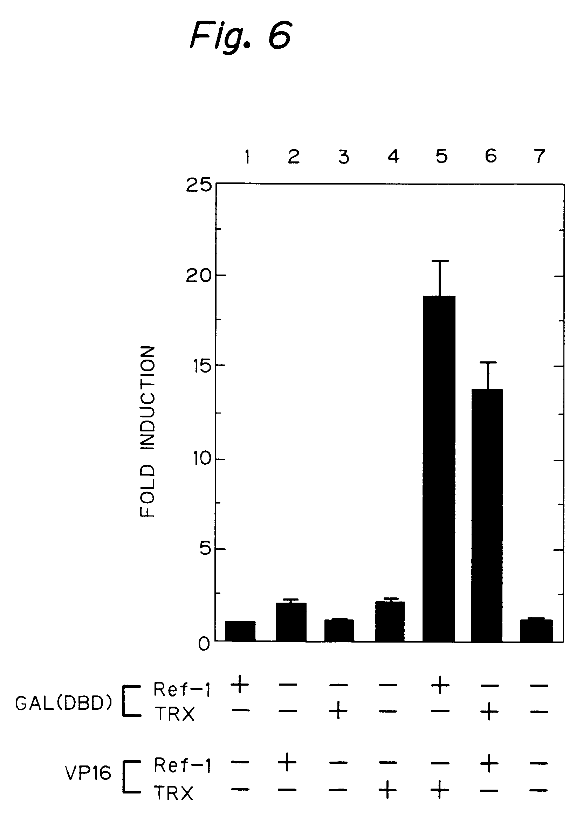

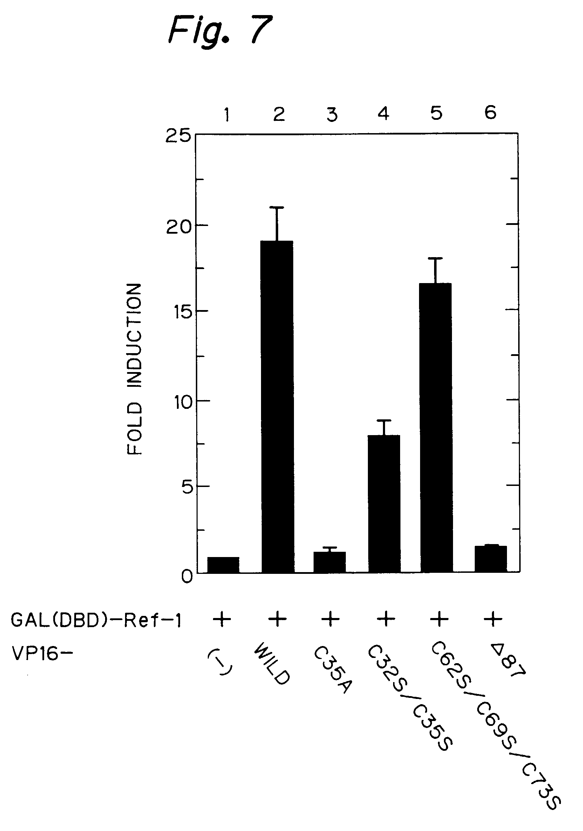

We also succeeded in detecting the association between TRX and Ref-1 in vivo by using mammalian two-hybrid assay (Example 4). If the expression of a reporter gene is detected by two-hybrid assay, it suggests that two fused proteins associate with each other in the nucleus of a living cell. More specifically, when the wild-type TRX and Ref-1 were co-expressed, the expression of a reporter gene showed a ten fold increase over the control (Fig. 6, lanes 5 and 6). The reporter gene expression level of TRX^{C62S/C69S/C73S} was almost the same as that of the wild-type. However, the reporter gene expression level of TRX^{C35A} did not reach a half of that of the wild-type (Fig. 7, lane 4) and no expression was induced by TRX^{C32S/C35S} (lane 3). Thus, the present invention also revealed in vivo that TRX and Ref-1 associate via Cys32 and Cys35 which constitute the active center of TRX, particularly Cys32.

Those skilled in the art will understand that active centers of TRX from other species may differ from the active center of human TRX, described above. An active center of TRX from a desired organism can easily be identified and modified by referring to the present specification.

We also prepared a mutant TRX Δ87 by deleting the C terminal of TRX (deleting the 88th to 104th amino acid residues of human TRX) and examined for its association with Ref-1 by the mammalian two-hybrid assay described above. As a result, no significant induction of a reporter gene was observed if a fusion construct of the TRX mutant lacking the COOH terminal and Ref-1 was co-expressed (Fig. 7, lane 6). This result suggests that the COOH terminal of TRX is indispensable for the reducing activity of TRX and the interaction between TRX and Ref-1.

As described above, it was first revealed that TRX and Ref-1 directly associate to promote the transcriptional activation of AP-1 and that said association requires Cys residues in the active site of TRX, such as Cys32 and Cys35, particularly Cys32 of human TRX. It is thus possible to obtain stable TRX which associates with Ref-1 under non-reducing conditions and does not form unnecessary S-S bonds to avoid multimer formation, by substituting Cys residues by other amino acid residues except that the Cys residues responsible for the direct association with Ref-1 are conserved. Therefore, the TRX variant of the present invention is characterized in that it is stable under non-reducing conditions by substituting at least one or all Cys residues by other amino acid residues except that Cys residues in the active center of TRX remain unmodified.

Necessary Cys residues for the activity and association of, for example, human TRX are Cys32 and Cys35. In the light of the description of the present specification, those skilled in the art can readily determine necessary Cys residues for the activity and association of non-human TRXs.

The TRX variant of the present invention can be obtained by conventional gene engineering technology. For example, the amino acid sequence and base sequence of TRX are known and described in a plurality of documents as mentioned above. Based on the prior art documents, cDNA encoding natural TRX can be obtained from an appropriate cDNA library. Then, a variant of the present invention can be obtained by, for example, a well-known technique, site-directed mutagenesis (for example, Nucleic Acid Research, Vol. 10, No. 20, pp. 6487-6500, 1982).

Site-directed mutagenesis method can be performed by using, for example, a synthetic oligonucleotide primer which is complementary to a single-stranded phage DNA to be mutated except for a specific mismatch corresponding to a desired mutation, as follows. Namely, said synthetic oligonucleotide is used as a primer to synthesize a strand which is complementary to the phage and the resulting double-stranded DNA is transformed into a host cell. Cultures of the transformed bacteria are plated onto agar to form plaques from a single cell containing the phage. Theoretically, 50 % of new colonies contain the single-stranded phage with mutation and the remaining 50 % has the original sequence. The resulting plaques are hybridized with a radioactively or otherwise labelled synthetic probe at a temperature which allows them to hybridize with DNA wholly identical with those having said desired mutation but not with DNA having the original strand. Then, the plaques which hybridized with said probe are collected and grown to harvest DNA.

The TRX variant of the present invention is characterized in that at least one or all Cys residues are substituted by other amino acid residues except that Cys residues in the active center remain unsubstituted. Thus, any deletion, addition or substitution of one or more other amino acid residues in the amino acid sequence of natural TRX may clearly be within the scope of the present invention so far as TRX can promote the AP-1 physiological activity and associate with Ref-1. The deletion, addition or substitution of these amino acids can be carried out by, for example, the site-directed mutagenesis method described above.

The present invention also provides a factor to enhance AP-1 transcriptional activity, wherein said factor comprises TRX variant and Ref-1 associated with each other via an S-S bond. The TRX variant in the AP-1 transcriptional activity enhancer of the present invention does not form a multimer and is stable under non-reducing conditions, because Cys residues outside the active site are substituted by other amino acid residues to avoid S-S bond formation.

The following examples illustrate the present invention, but are not intended to limit the technical scope thereof. Those skilled in the art can readily add modifications or changes to the present invention in the light of the description of the present specification, and they are also included in the technical scope of the present invention.

### EXAMPLES

The following materials and methods were used in the examples described below.

### Cells and Cell Culture

HeLa and COS7 cells were cultured in DMEM (Gibco BRL, Gaitherburg, USA) supplemented with 10 % fetal calf serum and antibiotics at 95 % humidity in 5 % CO₂ in air at 37°C.

### Reagents

12-O-tetradecanoylphorbol 13-acetate (PMA) was purchased from Sigma Chemicals Co., St. Louis, MO. Anti-TRX monoclonal antibodies 11-mAb and 21-mAb were produced and provided by Fujirebio Inc., Tokyo, Japan. Anti-Ref-1 polyclonal antibody was purchased from Santa Crutz Biotechnology, Inc., Palo Alto, USA.

### Mutagenesis of TRX and Transfection

Mutagenesis of human TRX was performed by a PCR-based technique and the sequences were verified by DNA sequencing (Fig. 1). The expression plasmids were introduced into cells by LipofectAMINE reagent (GIBCO/BRL, Gaithersburg, MD) following the manufacturer's instructions using Opti-MEM (GIBCO/BRL).

### Luciferase Assay Using -73/+63 Col (Human Collagenase I) -LUC

-73/+63 Col-LUC was constructed with pGL2-Basic (Promega) and -73/+63 Col in pBLCAT3 (Angel, P., Baumann, I., Stein, B., Delius, H., Rahmsdorf, H.J. & Herrlich, P. (1987) Mol. Cell Biol. 7, 2256-2266). HeLa cells were plated on 6-well plates at a density of 4 x 10⁵ cells per well. The expression plasmids were introduced into the cells using LipofectAMINE reagent. In each transfection, 1µg of the pCDSRα-TRX^{Wild} or -TRX^{C23S/C35S} expression plasmid (Tagaya, Y., Maeda, Y., Mitui, A., Kondo, N., Masutani, H., Mamuro, J., Brown, N., Arai, K., Yokota, T., Wakasugi, H. & Yodoi, J. (1989) EMBO J. 8, 757-764) and/or 1µg of the pRc/CMV-Ref-1 expression vector were used with 1µg of -73/+63 Col-LUC. The total amount of DNA was adjusted to 3µg by adding pRc/CMV (Invitrogen, San Diego, CA). After incubation for 16 h, PMA (30 ng/ml) was added and the cells were grown for 24 h. The cells were harvested after 30 h and luciferase activity was determined using a commercial assay system (Promega, Madison, WI) with a luminometer. The relative fold induction of luciferase activity was calculated.

### Bacterial Expression of Recombinant Proteins

Various TRX recombinant proteins fused with hexahistidine (6 x His) tag were expressed in E.coli using the pQE30 expression plasmid (Qiagen, Chatsworth, CA). The E.coli strain M15 [pRep4] was transformed with each pQE-TRX expression vector. Cells of E.coli were grown at 37°C with vigorous shaking until the A600 reached 0.7 and were treated for 3 h with 1mM IPTG. The pelleted cells were lysed in PBS(-) containing 1mg/ml lysozyme, 10mM 2-mercaptoethanol, 1mM PMSF, 10µM TLCK and 0.8mM imidazol and disrupted by sonication. The lysate was centrifuged to remove impurities. The crude lysate containing 6 x His-TRX was loaded onto an Ni²⁺-NTA-agarose column. The column was washed, and the fusion protein was eluted by PBS containing 80mM imidazol. The eluted protein was dialyzed against PBS containing 2mM DTT. Human Ref-1 was expressed as a 6 x His-tagged protein by pDS 56 ref-1 (Xanthoudakis, S., et al., 1994, supra.) (a gift from Xanthoudakis, S.) following the same protocol as described for TRX except that Ref-1 eluate was dialyzed against a storage butter [50mM sodium phosphate, pH 7.3 / 50mM NaCl / 5 mM MgCl₂ / 1mM EDTA, 5% (vol/vol) glycerol].

### In Vitro Disulfide Crosslinking Interaction Assay

6 x His-TRXs (200 ng) and 6 x His-Ref-1 (200 ng) were incubated for 30 min at room temperature (RT) with 1 mM DTT. After incubation, the reaction mixture was denatured for 5 min at 90°C in dissociation buffer with or without 2-ME (1 %). Each complex was electrophoresed on 15 % SDS-PAGE and transferred to PVDF membrane (Millipore, Bedford, MA), which was then blocked and reacted with antibodies followed by peroxidase-conjugated anti-mouse IgG (Amersham, Birmingham, UK). The antigens on the membrane were visualized with an ECL Western blot detection kit (Amersham).

### Indirect Immunofluorescence Cell Staining

Cells were immobilized on a slide in PBS containing 3.7% paraformaldehyde and 10% fetal calf serum for 20 min at room temperature (RT) followed by permeabilization for 10 min using (0.2% w/v) Triton X-100 in PBS. The slide was reacted with a primary antibody (monoclonal 11-mAb or 21-mAb for TRX, and the previously mentioned polyclonal antibody for Ref-1) for 1h at RT, and then with a secondary antibody (FITC-conjugated sheep anti-mouse IgG (Amersham Life Science Inc.) for TRX and TRITC-conjugated goat anti-rabbit IgG (Sigma Chemical Co.) for Ref-1) for 1 h. 90 % Glycerol containing 1 mg/ml p-phenylenediamine was applied on the slide carrying stained cells, and the cells were examined using a confocal microscope MRC 600 (Bio-Rad lab. Hercules, CA).

### Mammalian Two-Hybrid Assay

Mammalian two-hybrid assay was performed following the method described in Nerlov, C. & Ziff, E.B. (1995) EMBO J. 14, 4318-4328. A complementary DNA of TRX, TRX mutants or Ref-1 was fused in frame to pCMX-GAL4 (Sadowski, I. & Ptashne, M. (1989) Nucleic Acid Res. 17, 7539) or pCMX-VP16 (Perlmann, T., Rangarajan, P.N., Umesono, K. & Evans, R.M. (1993) Genes & Development 7, 1411-1422) (gifts from K. Umesono). COS7 cells were plated on 6-well plates at a density of 2 x 10⁵ cells per well. The expression plasmids were introduced into the cells by LipofectAMINE reagent. In each transfection, 1µg of GAL4-fused plasmid and 1µg of VP16-fused plasmid were used together with 1µg of a reporter construct ptk-GALpx3-LUC (a gift from K.U.) and 1µg of a β-gal expression construct as an internal control. The ptk-GALpx3-LUC contains 3 copies of GAL4-binding sites preceding a minimal thymidine-kinase promoter. The cells were harvested after 30 h and luciferase activity was determined. The relative fold induction of luciferase activity was calculated by normalization against β-gal activity.

### Example 1: Expression of TRX and/or Ref-1 in PMA-Stimulated HeLa Cells

We examined whether overexpressed TRX and/or Ref-1 resulted in transcriptional activation of AP-1 in vivo. We assayed the activity of the human collagenase I promoter, which contained an AP-1 binding site, by determining luciferase activity.

In each transformation, 1µg of the pCDSR α-TRX^{wild} (Fig. 2, lanes 2, 4) or -TRX^{C32S/C35S} (lanes 3, 6) expression plasmid and/or 1µg of the pRc/CMV-Ref-1 (lanes 1, 2, 3) expression vector were used with 1µg of -73/+63 Col-LUC. The total amount of DNA was adjusted to 3µg with pRc/CMV (Invitrogen, San Diego, CA). After incubation for 16 h, PMA (30 ng/ml) was added and the cells were grown for 24 h. The cells were harvested after 30 h and luciferase activity was determined.

The results are shown in Fig. 2. The results are the means of three experiments (each in duplicate) and presented as fold increases in luciferase activity over the baseline of PMA-untreated control (PMA-) seen with in lane 4.

Transient co-expression of the wild-type TRX and Ref-1 in PMA-treated HeLa cells (Fig. 2, lane 2) caused a 4-fold increase in luciferase activity over mock transfection (lane 4). Expression of either the wild-type TRX (lane 5) or Ref-1 (lane 1) alone resulted in a 1.5-fold or 2-fold activation, respectively. Expression of TRX^{C32S/C35S}, which lacked reducing activity, brought only marginal induction (lane 6).

Thus, overexpressed TRX and Ref-1 cooperatively enhance the transcriptional activity of AP-1.

### Example 2: Intracellular Translocation of TRX with PMA treatment

We examined intracellular localization of TRX and Ref-1. We analyzed HeLa cells with or without PMA treatment, wherein the transcriptional activation of AP-1 of said cells has been induced, by the indirect immunofluorescence method using antibodies raised against TRX or Ref-1 (Meyer, M., Schreck, R. & Baeuerle, P.A. (1993) EMBO J. 12, 2005-2015). Ref-1 was localized in nuclei and remained there after PMA stimulation. In contrast, TRX was localized predominantly in cytoplasms before PMA stimulation and then efficiently translocated into nuclei upon PMA treatment, and this process was almost completed in 24 h (with data not shown).

### Example 3: Associates between TRX and Ref-1 In Vitro

If an oxido-reductase directly interacts with its substrate, these molecules should form an intermediate through a disulfide bridge. Therefore, we reasoned that it would be possible to observe their transient association using a crosslinking reagent such as diamide, which converts free mercapto radicals to disulfides by cysteine oxidation.

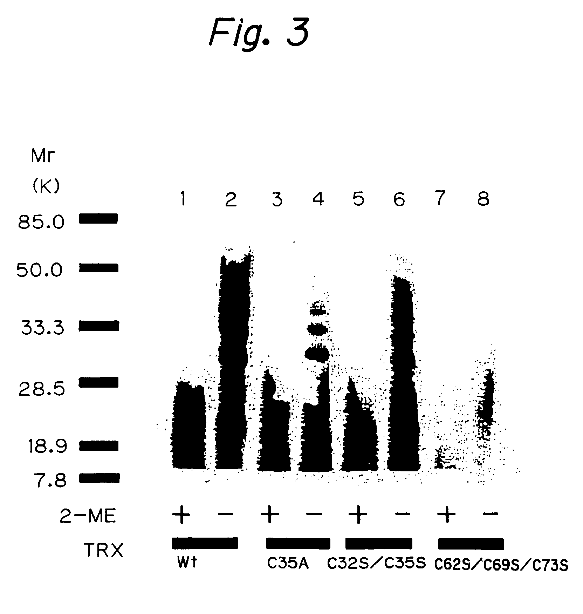

In order to determine which of the five cysteines in TRX is/are responsible for the intermolecular disulfide bond formation, three TRX mutants, which contained cysteines-to-serines or cysteine-to-alanine substitution(s), were purified as hexahistidine (6 x His)-tagged form. 6 x His-tagged Ref-1 and the various mutants of TRX (Fig. 1) were crosslinked with diamide in the presence or absence of Ref-1. After reaction, the complexes were separated by electrophoresis under reducing or oxidizing conditions and detected with antibodies. The results are shown in Figs. 3 to 5.

Under reducing conditions, the wild-type TRX and all the TRX mutants (TRX^{C32S/C35S}, TRX^{C35A} and TRX^{C62S/C69S/C73S}) migrated as a single 13-kDa band in SDS-PAGE. Treatment of the wild-type TRX or TRX^{C32S/C35S} with diamide generated multiple bands, suggesting multimer formation. In case of TRX^{C62S/C69S/C73S}, however, only a monomeric form was detected after diamide treatment (Fig. 3).

Ref-1 has seven cysteines, and migrated as a monomer under reducing conditions (Fig. 4, lanes 1, 3, 5, 7). Under oxidizing conditions, an additional band smaller than the non-reduced form was observed, representing probably a different monomeric form of Ref-1 with intramolecular disulfide bond (lanes 2, 4, 6, 8). Any further additional band was not found at higher molecular weights, suggesting that Ref-1 does not form any cysteine-mediated multimer under thiol-targeted oxidizing conditions.

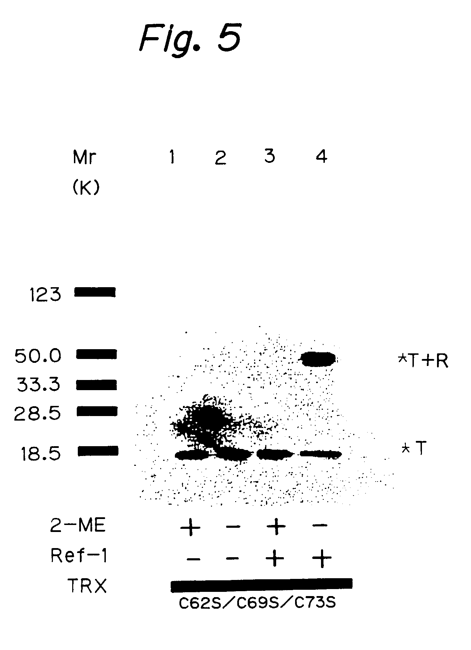

Next, the wild-type and mutant TRXs were incubated with Ref-1 and their migration patterns were analyzed. Under oxidizing conditions, both antibodies against Ref-1 (Fig. 4) and TRX (Fig. 5) recognized the same extra bands migrating around 50 kDa in lanes of the wild type TRX, TRX^{C35A} and TRX^{C62S/C69S/C73S} (Fig. 4, lanes 2, 4, 8; Fig. 5, T+R in lane 4), but did not recognize such a band in lane of TRX^{C32S/C35S} (Fig. 4, lane 6). These results showed that TRX and Ref-1 formed a covalent heterodimer in vitro and further suggested this association involved Cys 32 in the active center of TRX.

### Example 4: Physical Association between TRX and Ref-1 by Mammalian Two-Hybrid Assay

Mammalian two-hybrid assay is widely used to study interactions between proteins and especially useful for confirming interactions identified by two-hybrid library screening in yeast. This transient assay can also be used with deletional or site-directed mutagenesis to rapidly map the amino acids responsible for the interaction between two known proteins. If the expression of a reporter gene is detected by two-hybrid assay, it suggests that two fusion proteins associate with each other in the nucleus of a living cell.

We used mammalian two-hybrid assay to detect an association between TRX and Ref-1 in nuclei in vivo. A complementary DNA (cDNA) of TRX or Ref-1 was subcloned downstream of the DNA-binding domain (DBD) of GAL4 or the VP16 activation domain. These fusion plasmids were co-expressed in COS7 cells with a plasmid containing the luciferase gene as a reporter.

As shown in Fig. 6, no luciferase activity was observed (lanes 1, 3) when a DBD-TRX or DBD-Ref-1 was expressed with VP16 in COS7 cells. When VP16-TRX or VP16-Ref-1 was expressed with DBD, only marginal inductions were detected (lanes 2, 4). In contrast, co-expression of the wild-type TRX and Ref-1 fusion constructs induced luciferase activity (lanes 5, 6) more than 10 times higher than the basal level (lane 7).

Next, we tried to identify the cysteine residue(s) responsible for this interaction, using TRX mutants (Fig. 7). A triple mutation of non-catalytic cysteines (TRX^{C62S/C69S/C73S}) did not lose luciferase activity (lane 5), suggesting that these cysteines are dispensable for the activity of TRX to interact with Ref-1. Co-expression of a fusion construct of TRX^{C35A} and Ref-1 induced luciferase activity (lane 4) about 6 to 7 times higher than the basal level (lane 1). On the other hand, co-expression of a fusion construct of TRX^{C32S/C35S} and Ref-1 brought no induction of luciferase (lane 3).

These in vitro (Example 3) and in vivo (Example 4) data showed that a direct physical interaction between TRX and Ref-1 was mediated by Cys32 and Cys35 in the active center.

In Figs. 6 and 7, relative induced luciferase was calculated by normalization against β-gal activity. The results are the means of three experiments (each in duplicate) and presented as fold increases in luciferase activity over the baseline seen with lane 1.

In addition to the substitution mutants, we constructed a truncated TRX, TRX Δ87 (Fig. 1) by deleting a C-terminal domain including the 88th and succeeding amino acids residues. TRX Δ87 conserves all the five cysteines but loses its dithiol reducing activity. Co-expression of a fusion construct of TRX Δ87 and Ref-1 showed no significant induction of luciferase activity (Fig. 7, lane 6). This result suggests that the reducing activity of TRX and the interaction between TRX and Ref-1 require not only Cys residues in the active site but also the COOH terminal of TRX.

The present invention has thus provided TRX which is stable under non-reducing conditions and an AP-1 transctiptional activity enhancer wherein said TRX and Ref-1 are associated via an S-S bond. The enhancer of the present invention promotes the transcriptional activity of AP-1 by S-S linkage between TRX and Ref-1 at the active site of TRX.

## Claims

1. A thioredoxin variant which has been made stable under non-reducing conditions, by substituting at least one or all Cys residues by other amino acid residues except that Cys residues in the active center of thioredoxin remain unmodified.

2. A factor for enhancing the transcriptional activity of AP-1 wherein the thioredoxin according to Claim 1 and Ref-1 are associated with each other via an S-S bond.
